(19) Europäisches Patentamt
European Patent Office
Office européen des brevets

(11) Veröffentlichungsnummer: **0 467 308 A2**

(12) # EUROPÄISCHE PATENTANMELDUNG

(21) Anmeldenummer: **91111871.9**

(51) Int. Cl.5: **C07D 213/64**

(22) Anmeldetag: **16.07.91**

(30) Priorität: **17.07.90 CH 2373/90**

(43) Veröffentlichungstag der Anmeldung:
**22.01.92 Patentblatt 92/04**

(84) Benannte Vertragsstaaten:
**AT BE CH DE DK ES FR GB IT LI NL SE**

(71) Anmelder: **LONZA AG**
**Gampel/Wallis Geschäftsleitung Basel**
**CH-4002 Basel(CH)**

(72) Erfinder: **Worsch, Detlev, Dr.**
**Faxugrundstrasse 30**
**Brigerbad (Kanton Wallis)(CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

(54) **Verfahren zur Herstellung von 2-Hydroxy-3-halogen-5-nitropyridinen.**

(57) Es wird ein neues Verfahren zur Herstellung von 2-Hydroxy-3-halogen-5-nitropyridinen beschrieben, wobei eine 5-Halogen-6-hydroxynikotinsäure zum Endprodukt nitriert wird.
Die resultierenden Pyridine bilden wertvolle Zwischenprodukte für Wirkstoffsynthesen.

EP 0 467 308 A2

Die Erfindung betrifft ein neues Verfahren zur Herstellung von 2-Hydroxy-3-halogen-5-nitropyridinen.

Diese Verbindungen sind dank der reaktionsfähigen funktionellen Gruppen universell für Wirkstoffsynthesen für Pharmazeutika oder Herbizide anwendbar.

Beispielsweise kann das gemäss CA 70: 106327x (T.Batkowski, Rocz.Chem. 42 (2) 1968, S.2079-2088) durch Chlorierung gewonnene 2,3-Dichlor-5-nitropyridin gemäss DE-OS 35 45 570 zur Synthese von Herbiziden eingesetzt werden.

Gemäss CA 70: 106327x (T.Batkowski, Rocz.Chem. 42 (12) 1968, S.2079-2088) war es bekannt, das 2-Hydroxy-3-chlor-5-nitropyridin, ausgehend von 2-Amino-5-nitropyridin, durch Chlorierung zum 2-Amino-3-chlor-5-nitropyridin (Ausbeute 28%) und durch anschliessende Diazotierung/Verseifung der Aminogruppe (Ausbeute 72%) herzustellen.

Die ungenügenden Ausbeuten sowie das schwer verfügbare Edukt 2-Amino-3-chlor-5-nitropyridin gaben dazu Anlass, ein neues Verfahren zur Herstellung der 2-Hydroxy-3-halogen-5-nitropyridine zu entwickeln.

Diese Aufgabe konnte gelöst werden mit einem Verfahren gemäss Anspruch 1.

Erfindungsgemäss wird dabei eine 5-Halogen-6-hydroxynikotinsäure der allgemeinen Formel

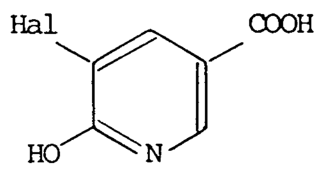

II

worin Hal Chlor, Brom oder Jod bedeutet, in Gegenwart von Salpetersäure und Schwefelsäure zu den 2-Hydroxy-3-halogen-5-nitropyridinen der allgemeinen Formel

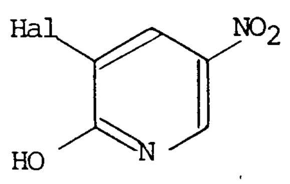

I

worin Hal die genannte Bedeutung hat, nitriert.

Die 5-Halogen-6-hydroxynikotinsäure, vorzugsweise die 5-Chlor-6-hydroxynikotinsäure, kann auf einfache Weise aus der grosstechnisch verfügbaren 6-Hydroxynikotinsäure durch Umsetzung mit einem Säurehalogenid und anschliessender Hydrolyse des entstehenden 5-Halogen-6-hydroxynikotinsäurehalogenids gemäss CH-PS 664 754 hergestellt werden.

Die erfindungsgemässe Umsetzung erfolgt zweckmässig bei Temperaturen zwischen 0 und 100 °C, vorzugsweise zwischen 40 und 55 °C.

Zweckmässig gelangen Mischungen von konzentrierter Salpetersäure und konzentrierter Schwefelsäure im Verhältnis von 4 zu 1, vorzugsweise im Verhältnis von 1 zu 1 zum Einsatz. Mischungen mit weniger Schwefelsäure können aber ebenfalls eingesetzt werden.

Nach einer Reaktionszeit von ca. 1 bis 3 h kann das Reaktionsgemisch auf übliche Weise, vorzugsweise durch Aufnehmen in Eiswasser, durch Filtrieren der entstehenden Suspension und Trocknen des Filtergutes, aufgearbeitet werden.

Die resultierenden 2-Hydroxy-3-halogen-5-nitropyridine können auf diese Weise in hoher Reinheit und guter Ausbeute erhalten werden.

## Beispiel

200 ml Salpetersäure (70%) wurden vorgelegt und auf 5 °C abgekühlt. 200 ml konz. Schwefelsäure wurden unter Rühren bei 5-10 °C langsam zugegeben. Anschliessend wurden bei 5 °C 100 g (0,576 mol) 5-Chlor-6-hydroxynikotinsäure langsam eingetragen. Das Gemisch wurde auf 50 °C erwärmt.

Nach Abklingen der exothermen Reaktion liess man noch 2 h bei 50 °C stehen und kühlte dann auf Raumtemperatur ab.

Das Gemisch wurde in 1,5 1 Eiswasser gegossen und die erhaltene Suspension auf -10 °C abgekühlt.

Man saugte ab, wusch den Filterrückstand mit Wasser pH-neutral und trocknete im Vakuum über Nacht.

Man erhielt 74,4 g eines blassgelben Pulvers. Ausbeute 74%.
$^{1}$N-NMR: (DMSO $d_6$, 300 MHz) $\delta$ in ppm
8,45 (d, 3 Hz, Ar-H)
8,72 (d, 3 Hz, Ar-H)
13,25 (breit, -OH)

| Elementaranalyse für $C_5H_3N_2O_3Cl$ (174,54) | | | |
|---|---|---|---|
| ber. | C 34,4% | H 1,7% | N 16,1% |
| gef. | C 34,7% | H 1,6% | N 16,5% |

**Patentansprüche**

**1.** Verfahren zur Herstellung von 2-Hydroxy-3-halogen-5-nitropyridinen der allgemeinen Formel

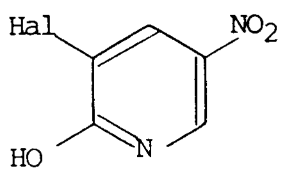

I

worin Hal Chlor, Brom oder Jod bedeutet, dadurch gekennzeichnet, dass eine 5-Halogen-6-hydroxynikotinsäure der allgemeinen Formel

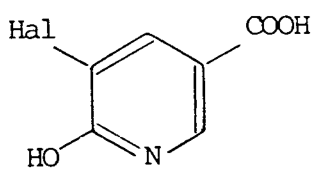

II

in Gegenwart von Salpetersäure und Schwefelsäure nitriert wird.

**2.** Verfahren nach Patentanspruch 1, dadurch gekennzeichnet, dass die Nitrierung bei Temperaturen zwischen 0 und 100 °C durchgeführt wird.

**3.** Verfahren nach mindestens einem der Patentansprüche 1 und 2, dadurch gekennzeichnet, dass die Nitrierung mit einer Mischung von konzentrierter Salpetersäure und von konzentrierter Schwefelsäure in einem Verhältnis von 4 zu 1 bis 1 zu 1 durchgeführt wird.